# EUROPEAN PATENT SPECIFICATION

(11) **EP 2 110 088 B1**
(45) Date of publication and mention of the grant of the patent: **20.06.2012**
(21) Application number: 09251088.2
(22) Date of filing: 14.04.2009
(51) Int. Cl.: A61B 17/34, A61M 39/06

(54) **Self-conforming surgical seal**
Selbstanpassendes chirurgisches Siegel
Joint chirurgical auto-conformable

(30) Priority: 15.04.2008 US 44955 P; 10.03.2009 US 400842
(43) Date of publication of application: 21.10.2009
(73) Proprietor: Tyco Healthcare Group LP, New Haven, CT 06511 (US)
(72) Inventor: Okoniewski, Gregory G., North Haven, CT 06473 (US)
(74) Representative: Soames, Candida Jane

(56) References cited:
- EP-A- 1 348 386
- WO-A-94/17844
- US-A- 5 779 697
- US-A1- 2002 183 594

## Description

### BACKGROUND

### 1. Technical Field

The present disclosure relates to a surgical seal of the type adapted for the sealed reception of a surgical object. In particular, this disclosure relates to a surgical seal for use with a surgical access member such as a cannula or trocar assembly.

### 2. Background of the Related Art

Many contemporary medical and surgical procedures are performed through access members. These devices incorporate narrow tubes or cannulas percutaneously inserted into a patient's body and have a central opening through which surgical objects are introduced and manipulated during the course of the procedure. Generally, such procedures are referred to as "endoscopic", and, if performed on the patient's abdomen, the procedure is referred to as "laparoscopic". Throughout the present disclosure, the term "minimally invasive" should be understood to encompass both endoscopic and laparoscopic procedures.

Generally, during minimally invasive procedures, prior to the introduction of a surgical object into the patient's body, insufflation gases are used to enlarge the area surrounding the target surgical site to create a larger, more accessible workspace. Accordingly, the maintenance of a substantially fluid-tight seal along the central opening of the access member, in both the presence and absence of a surgical object, is desirable so as to prevent the escape of the insufflation gases and the deflation or collapse of the enlarged surgical workspace. To this end, surgical access members generally incorporate one or more seals many varieties of which are known in the art. One such example may be seen in commonly assigned U.S. Patent No. 5,512,053 to Pearson. Other examples are EP 1 348 386 A1 and US 5,779,697 with reinforced seals, EP 1 348 386 A1 forming the basis for the preamble of claim 1.

During the course of a minimally invasive procedure, a clinician will frequently move surgical objects laterally within the access member, and the seal disposed therein, to access different regions of the surgical workspace. This lateral movement may distort the seal, thereby potentially causing the escape of insufflation gas and compromising the integrity of the insufflated workspace.

While many varieties of surgical seals are known in the art, a continuing need exists for a seal capable of maintaining the integrity of an insufflated workspace during lateral movement of a surgical object inserted therethrough.

### SUMMARY

Accordingly, the present disclosure is directed to a surgical access apparatus, including an access member defining a longitudinal axis and having a longitudinal passageway for reception and passage of a surgical object, a seal member mounted to the access member and having inner seal portions defining an aperture to removably receive the surgical object in substantial fluid-tight sealing relation therewith and at least one cable member. The at least one cable member has a first cable end connected to a first radial section of the seal member and a second cable end connected to a second radial section of the seal member displaced from the first radial section wherein the first and second ends are in substantially diametrically opposed relation. The first and second cable ends are adapted to be laterally displaced relative to the longitudinal axis during offset lateral movement of the surgical object, to thereby cause corresponding lateral displacement of the inner seal portions of the seal member whereby the inner seal portions maintain the substantial fluid tight sealing relation with the surgical object. The first and second cable ends may be connected to respective first and second radial sections of the seal member at locations adjacent the inner seal portions. The first and second radial sections may be in general diametrically opposed relation. First and second cable members may be provided with each cable member having cable ends connected to spaced radial sections of the seal member.

The first cable end may be connected to the first radial section at a first location and the second cable end may be connected to the second radial section at a second location with the first and second locations being substantially adjacent the aperture.

The seal member may define at least one channel configured to at least partially receive the at least one cable member. The at least one channel may be defined within the seal member. Alternatively, the at least one channel is defined on an outer surface of the seal member.

The at least one cable member may define a length that remains substantially constant during use of the surgical seal member. The at least one cable member may be formed of a substantially non-rigid material.

The seal member may be formed of an elastomeric material such that the seal member resiliently transitions between first and second conditions upon the respective insertion and removal of the surgical object. The aperture of the seal member may define a first diameter in the first condition and a second diameter in the second condition. The at least one cable member is configured to displace the aperture of the seal member upon lateral manipulation of the surgical object inserted therethrough with the aperture being displaced in the direction of lateral manipulation such that the second diameter of the aperture remains substantially constant.

The access member may include a cannula defining a longitudinal opening dimensioned for passage of the surgical object. A housing may be coupled to the cannula. The housing is configured to receive the seal member and defines at least one cable conduit. The at least one cable conduit is configured to permit displacement of the at least one cable member during lateral manipulation of the surgical object within the seal member.

These and other features of the surgical seal disclosed herein will become more readily apparent to those skilled in the art from the following detailed description of various embodiments of the present disclosure.

### BRIEF DESCRIPTION OF THE DRAWINGS

Various embodiments of the present disclosure are described hereinbelow with references to the drawings, wherein:

**FIG. 1A** is a top perspective view of a seal member in accordance with the principles of the present disclosure.

**FIG. 1B** is a top perspective view of one embodiment of the seal member of **FIG. 1A****.**

**FIG. 2A** is a top plan view of the seal member of **FIG. 1A** shown in a first condition.

**FIG. 2B** is a top perspective view of the seal member of **FIG. 1A** shown in a second condition with a surgical object inserted therethrough.

**FIG. 2C** is a top plan view of the seal member of **FIG. 1A** with first and second cable members.

**FIG. 3A** is a top perspective view of another embodiment of the seal member of FIG.1 including channels defined in the proximal surface thereof.

**FIG. 3B** is a side plan view of another embodiment of the seal member of **FIG. 1** including a channel defined in the periphery thereof.

**FIG. 3C** is a top plan view of another embodiment of the seal member of **FIG. 1** including channels defined within the seal member.

**FIG. 3D** is a side plan view of the seal member of **FIG. 3C**;

**FIG. 4A** is a perspective view with parts separated of a surgical access member for use in conjunction with the seal member of **FIG. 1**.

**FIG. 4B** is side cross-sectional view of the housing of the surgical access member taken along line **4B-4B** of **FIG. 4A**.

**FIG. 5** is a top perspective view of the seal member of **FIG. 1** illustrating the forces exerted thereupon by a surgical object following insertion.

**FIG. 6** is a top plan view of a known, exemplary seal member upon lateral manipulation of a surgical object inserted therethrough.

**FIG. 7** is a top plan view of the seal member of **FIG. 5** upon lateral manipulation of the surgical object.

### DETAILED DESCRIPTION OF THE EMBODIMENTS

In the drawings and in the description which follows, in which like reference numerals identify similar or identical elements, the term "proximal" will refer to the end of the apparatus closest to a clinician during the use thereof, while the term "distal" will refer to the end which is furthest from the clinician, as is traditional and known in the art.

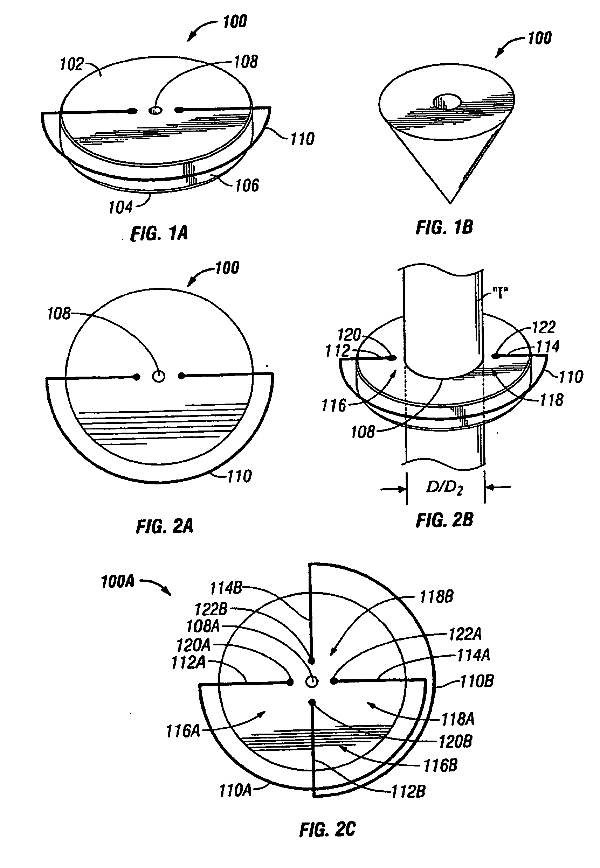

With reference to **FIGS. 1A-1B****.** a seal member **100** is disclosed that defines respective proximal and distal surfaces **102, 104,** a periphery **106** and an aperture **108** that is configured to removably receive a surgical object "I" **(****FIG. 2B****)** such that a substantially fluid-tight seal is formed therewith. Seal member **100** includes at least one cable member **110** which is discussed in detail below.

Seal member **100** may exhibit any configuration suitable for the intended purpose of receiving surgical object "**I**" so as to form a substantially fluid-tight seal therewith, including but not being limited to a substantially planar configuration, as seen in **FIGS. 1A**, or a generally conical configuration, as seen in **FIG. 1B****.**

Seal member **100** may be formed of any suitable biocompatible material that is at least semi-resilient in nature, including but not limited to elastomeric materials. Forming seal member **100** of such a material facilitates the resilient deformation of seal member **100,** and aperture **108** in particular, upon the insertion and removal of surgical object "I". The resilient nature of seal member **100** allows seal member **100** to exhibit various degrees of deformation during use, thereby facilitating the accommodation of surgical objects of various sizes, as well as the maintenance of a substantially fluid-tight seal therewith during the axial or lateral manipulation thereof within seal member **100**, as discussed in further detail below.

As seen in **FIGS. 2A-2B****.** prior to receiving surgical object "I", seal member **100** is in a first condition in which aperture **108** of seal member **100** defines a first diameter **"D₁"** that is substantially less than the diameter "**D**" of surgical object "I". Aperture **108** may be closed in the first position, i.e., such that "**D₁**" equals zero, to thereby prevent the escape of any insufflation gas through seal member **100** in the absence of surgical object "I". Upon the insertion of surgical object **"I"**, aperture **108** deforms, or stretches, to accommodate the larger diameter **"D"** of surgical object **"I"**, thereby transitioning into a second condition. In the second condition, aperture **108** of seal member **100** defines a second diameter **"D₂"** that substantially approximates the diameter **"D"** of surgical object **"I"**, thereby forming a substantially fluid-tight seal with surgical object **"I"** and substantially preventing the escape of insufflation gas. The diameter **"D"** of the surgical object **"I"**, and thus the diameter **"D₂"** of the aperture **108** of seal member **100** in the second condition, will generally lie within the range of about 5mm to about 15mm, as is conventional in the art, although substantially greater and lesser values for diameter **"D₂"** are also within the scope of the present disclosure.

Referring still to **FIGS. 2A-2B****.** cable member **110** of seal member **100** will be discussed.

The cable member, or members, **110** may be formed of any suitable biocompatible material that is substantially non-rigid and substantially non-extensible in character, e.g. stainless steel, polymeric material, etc., such that the length of cable member **110** remains substantially constant during the use of seal member **100.** Cable member **110** has respective first and second ends **112**, **114** that are attached to seal member **100** at respective first and second sections **116**, **118** thereof. The first and second ends **112, 114** of cable member **110** are attached to the first and second sections **116**, **118** at first and second locations **120, 122**, respectively, that are disposed substantially adjacent to aperture **108** and spaced apart from one another. Various arrangements for securing the first and second ends **112**, **114** of the cable member **110** are envisioned. For example, seal member **100** may have posts embedded within the material of the seal member **100.** The first and second ends **112**, **114** may be attached or secured to the posts. In the alternative, the first and second ends **112**, **114** may be embedded within the seal member **100** during manufacture of the seal member **100**, such as, for example, during a molding process. Other means for attaching the first and second ends **112**, **114** are also envisioned. The respective first and second sections **116**, **118**, and consequently the respective first and second locations **120**, **122**, are in substantially diametric opposition to each other.

With reference now to **FIG. 2C**, in one embodiment, a seal member **100_{A}** is disclosed that includes a first cable member **110_{A}** having respective first and second ends **112_{A}, 114_{A}** attached to first and second sections **116_{A}**, **118_{A}** of seal member **100_{A}** at first and second locations **120_{A}**, **122_{A}**, respectively. In this embodiment, seal member **100_{A}** further includes a second cable member **110_{B}** having respective first and second ends **112_{B}, 114_{B}** attached to first and second sections **116_{B}, 118_{B}** of seal member **100_{A}** at first and second locations **120_{B}, 122_{B},** respectively. As with seal member **100** of **FIGS. 2A-2B****.** each of the first and second locations **120_{A}**, **122_{A}**, **120_{B}**, **122_{B}** are disposed substantially adjacent aperture **108_{A}** and spaced apart from one another. The incorporation of additional cable members, e.g., a second cable member **110_{B},** or three or more cable members, facilitates more uniform deformation of aperture **108_{A}** upon laterally manipulating a surgical object "I" **(****FIG. 2B****)** inserted therethrough, as discussed below.

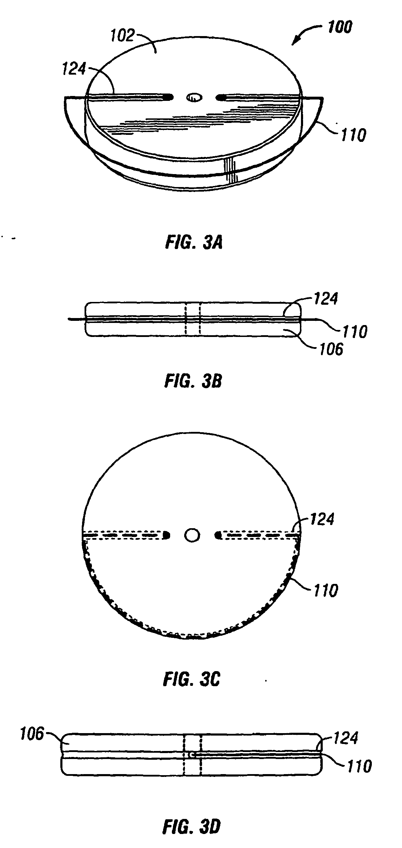

As seen in **FIGS. 3A-3D****,** in one embodiment, seal member **100** defines at least one channel **124** configured to at least partially receive cable member **110**. Channels **124** may be formed either in an outer surface of seal member **100**, e.g. proximal surface **102 (****FIG. 3A****),** distal surface **104** (not shown) or periphery 106 **(****FIG. 3B****)**, or within seal member **100 (****FIGS. 3C-3D****)** such that cable member **110** is at least partially concealed by seal member **100.**

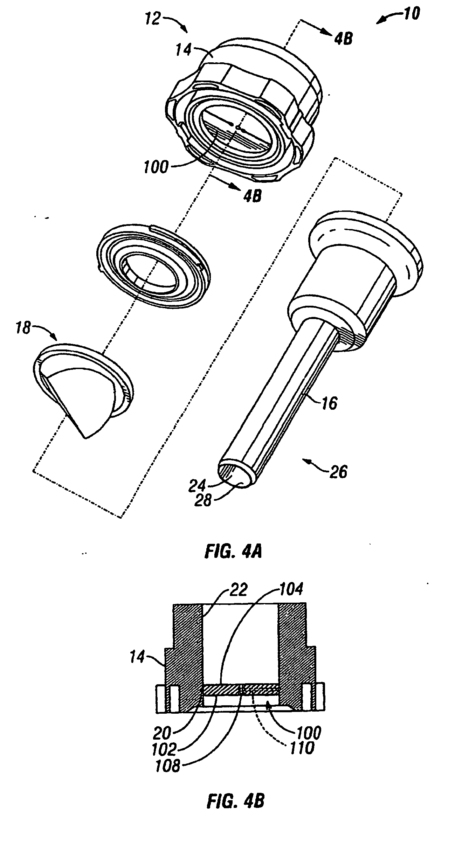

Referring now to **FIG. 4A**, a surgical access member, in the form of, e.g., a cannula assembly **10**, is illustrated that may be used in conjunction with seal member **100.** At a proximal end **12**, cannula assembly **10** includes a housing **14** that is configured to accommodate the seal **100** that is the subject of the present disclosure. Extending distally from housing **14** is a cannula or elongate member **16**. As illustrated, cannula assembly **10** may optionally further include a zero-closure valve **18**.

Housing **14** may be any structure suitable for the intended purpose of accommodating seal member **100**. As seen in **FIG. 4B**, in one embodiment, housing **14** defines at least one conduit **20** on an internal surface **22** thereof. Conduit **20** is configured to receive cable member **110** and to permit the displacement thereof during lateral manipulation of surgical object "I" within seal member **100**, as discussed in further detail below. Further information regarding seal housing **14** may be obtained through reference to commonly owned U.S. Patent No. 7,169,130 to Exline et al..

Cannula 16 extends distally from housing **14** and defines a longitudinal passage **24** that is configured to permit a surgical object "**I**" **(****FIG. 2B****)**, to pass therethrough, e.g., an obturator, trocar or endoscope. At its distal end **26**, cannula **16** defines an opening **28** that is configured to allow the surgical object "**I**" to pass therethrough. Conventionally, surgical objects generally define a diameter substantially within the range of about 3mm to about 15mm. Accordingly, longitudinal passage **24** will be dimensioned similarly, although substantially larger and smaller surgical objects and a cannula **16** defining a substantially larger or smaller longitudinal passage **24** and opening **28** are also within the scope of the present disclosure.

Referring now to **FIGS. 2A****,** **4A** and 5-7, the use and function of seal member **100** will be described in conjunction with a surgical access apparatus, e.g., cannula assembly **10.** Initially, the target surgical site is insufflated with a suitable biocompatible gas, e.g., CO₂ gas, such that a larger internal workspace may be created within a patient, thereby providing greater access to the patient's internal organs and/or cavities. The insufflation may be performed with an insufflation needle or similar device, as is conventional in the art. Thereafter, a variety of surgical objects, depicted generally as surgical object **"I"**, are inserted into cannula assembly **10** and advanced distally through seal member **100** and elongate member **16** to percutaneously access the insufflated workspace and carryout the minimally invasive procedure.

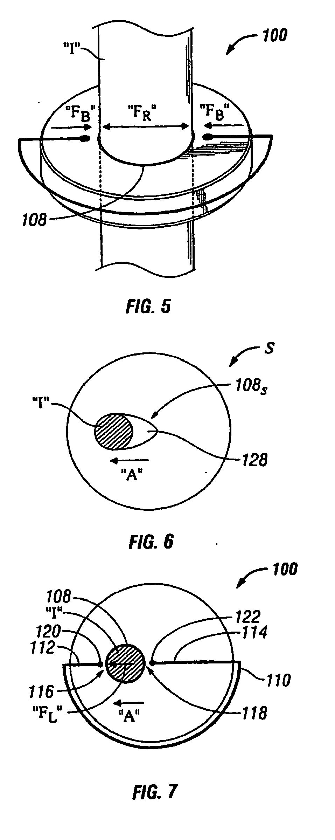

Subsequent to insufflation, seal member **100** substantially prevents the escape of insufflation gas, thereby maintaining the integrity of the insufflated workspace in both the absence and presence of surgical object **"I".** As seen in **FIG. 2A****,** prior to the insertion of surgical object **"I"**, seal member **100** is in the first condition, in which aperture 108 defines a first diameter **"D₁".** Upon the insertion of surgical object "I" **(****FIG. 5****)**, seal member **100,** and in particular the aperture **108** thereof, is subjected to a force **"F_{R}"** applied by surgical object **"I"** that is directed radially outward. Force **"F_{R}"** forces open aperture **108,** thereby transitioning seal member **100** into the second condition thereof in which aperture **108** defines a second, larger diameter **"D₂"** that substantially approximates the diameter **"D"** of surgical object **"I".** In the second condition, aperture **108** exerts a biasing force **"F_{B}"** directed radially inward that attempts to return seal member **100** to the first condition. Biasing force **"F_{B}"** is exerted upon surgical object **"I"**, thereby creating a substantially fluid-tight seal therewith.

As previously discussed, it is often necessary to axially or laterally manipulate surgical object during the course of a minimally invasive procedure to access different areas of a surgical workspace. **FIG. 6** describes the impact of such lateral manipulation upon a known seal **"S"**. As would be appreciated by one of ordinary skill, laterally manipulating surgical object **"I"** in the direction of arrow **"A"** can laterally distort the enlarged aperture **108s** of the seal **"S",** thereby creating a leak path **128** and potentially resulting in the escape insufflation gas therethrough. Seal member **100** of the present disclosure mitigates this potentiality through the incorporation cable member, or members, **110.**

As seen in **FIG. 7****,** upon the lateral movement of surgical object **"I"** in the direction of arrow **"A"**, a force **"F_{L}"** is applied to seal member **100** at the first section 120 thereof. Force "F_{L}" attempts to distort aperture **108** in the direction of arrow **"A"** and thereby create a leak path **128 (****FIG. 6****)**. Upon the application of force **"F_{L}"** to the first section **116,** the first end **112** of cable member **110** is subjected to force **"F_{L}"** through the connection between the first end **112** of cable member **110** and seal member **100** at first location **120.** Force **"F_{L}"** displaces the first section **116**, thereby displacing the first end **112** of cable member **110,** and ultimately the second end **114** thereof. Through the connection between the second end **114** of cable member **110** and seal member **100** at the second location **122,** the second section **118** of seal member **100** is subjected to the influence of force **"F_{L}"** and is also displaced in the direction indicated by arrow **"A"**. Consequently, aperture **108** deforms in a substantially uniform manner, maintaining its diameter **D₂** in the second condition and minimizing the dimensions of leak path **128,** if any, such that the substantially fluid-tight seal formed with surgical object **"I"** is preserved and the escape of insufflation gas through seal member **100** is curtailed. The incorporation of additional cable members **(****FIG. 2C****)** further ensures uniform deformation of aperture **108** upon the lateral movement of surgical object **"I"** and the preservation of a substantially fluid-tight seal therewith. The present disclosure contemplates that that material comprising cable member **110**, and the configuration and dimensions thereof, may be such that the degree of distortion realized by the second section **118** of the seal member **100** will approximate that of the first section **116**, thereby substantially maintaining the diameter **"D₂"** of aperture **108** in the second condition during the lateral manipulation of surgical object **"I".**

Although the illustrative embodiments of the present disclosure have been described herein with reference to the accompanying drawings, the above description, disclosure, and figures should not be construed as limiting, but merely as exemplifications of particular embodiments. It is to be understood, therefore, that the disclosure is not limited to those precise embodiments, and that various other changes and modifications may be effected therein by one skilled in the art without departing from the scope of the invention as defined by appended claims.

## Claims

1. A surgical access apparatus for a surgical object, which comprises:
an access member (10) defining a longitudinal axis and having a longitudinal passageway for reception and passage of a surgical object (I);
a seal member (100) mounted to the access member (10), the seal member (110) having inner seal portions defining an aperture (108) to removably receive the surgical object (I) in substantial fluid-tight sealing relation therewith; and **characterized by**
at least one cable member (110), the at least one cable member (110) having a first cable end (112) connected to a first radial section (116) of the seal member (100) and a second cable end (114) connected to a second radial section (118) of the seal member (110) displaced from the first radial section (116), wherein the first and second ends are in substantially diametrically opposed relation, the first and second cable ends (112, 114) adapted to be laterally displaced relative to the longitudinal axis during offset lateral movement of the surgical object, to thereby cause corresponding lateral displacement of the inner seal portions of the seal member (100) whereby the inner seal portions maintain the substantial fluid tight sealing relation with the surgical object (I).

2. The surgical access apparatus of claim 1, wherein the first and second cable ends (112, 114) are connected to respective first and second radial sections (116, 118) of the seal member (100) at locations (120, 122) adjacent the inner seal portions.

3. The surgical access apparatus of claim 2 including first and second cable members (110A, 110B), each cable member (110A, 110B) having cable ends (112A, 112B, 114A, 114B) connected to spaced radial sections (116A, 116B, 118A, 118B) of the seal member (100).

4. The surgical access apparatus of claim 1, wherein the first cable end (112) is connected to the first radial section (116) at a first location (120) and the second cable end (114) is connected to the second radial section (118) at a second location (122), the first and second locations (120, 122) being substantially adjacent the aperture (108).

5. The surgical access apparatus of claim 1, wherein the seal member (100) defines at least one channel (124) configured to at least partially receive the at least one cable member (110).

6. The surgical access apparatus of claim 5, wherein the at least one channel (124) is defined within the seal member (100).

7. The surgical access apparatus of claim 6, wherein the at least one channel (124) is defined on an outer surface (106) the seal member (100).

8. The surgical access apparatus of claim 1, wherein the at least one cable member (110) defines a length that remains substantially constant during use of the surgical seal member (110).

9. The surgical access apparatus of claim 1, wherein the at least one cable member (110) is formed of a substantially non-rigid material.

10. The surgical access apparatus of claim 1, wherein the seal member (100) is formed of an elastomeric material such that the seal member resiliently transitions between first and second conditions upon the respective insertion and removal of the surgical object.

11. The surgical access apparatus of claim 1, wherein the aperture (108) of the seal member (100) defines a first diameter (D₁) in the first condition and a second diameter (D₂) in the second condition.

12. The surgical access apparatus of claim 11, wherein the at least one cable member (110) is configured to displace the aperture (108) of the seal member (100) upon lateral manipulation of the surgical object (I) inserted therethrough, the aperture (108) being displaced in the direction of lateral manipulation such that the second diameter (D₂) of the aperture (108) remains substantially constant.

13. The surgical access apparatus according to claim 1, wherein the access member (10) includes a cannula (16) defining a longitudinal opening dimensioned for passage of the surgical object.

14. The surgical access apparatus according to claim 13, further including a housing (14) coupled to the cannula, the housing being configured to receive the seal member (100) and defining at least one cable conduit (20), the at least one cable conduit (20) being configured to permit displacement of the at least one cable member (110) during lateral manipulation of the surgical object within the seal member (110).

## Patentansprüche

1. Chirurgisches Zugangsgerät für einen chirurgischen Gegenstand, umfassend:
ein Zugangselement (10), das eine Längsachse definiert und einen Längsdurchgang für die Aufnahme und den Durchgang eines chirurgischen Gegenstands (I) aufweist,
ein Siegelelement (100), das auf das Zugangselement (10) montiert ist, wobei das Siegelelement (100) innere Siegelabschnitte aufweist, die eine Öffnung (108) definieren, um das chirurgische Element (I) in einer im Wesentlichen flüssigkeitsdichten Beziehung damit entfernbar aufzunehmen; und **gekennzeichnet durch**
mindestens ein Kabelelement (110), wobei das mindestens eine Kabelelement (110) ein erstes Kabelende (112) aufweist, das mit einem ersten radialen Abschnitt (116) des Siegelelements (100) verbunden ist, und ein zweites Kabelende (114), das mit einem zweiten radialen Abschnitt (118) des Siegelelernents (100), entfernt vom ersten radialen Abschnitt (116), verbunden ist, wobei das erste und das zweite Ende in einer im Wesentlichen diametral entgegengesetzten Beziehung zueinander stehen, wobei das erste und das zweite Kabelende (112, 114) angepasst sind, um seitlich bezüglich der Längsachse während der versetzen seitlichen Bewegung des chirurgischen Gegenstands verschoben zu werden, um **dadurch** die entsprechende seitliche Bewegung der inneren Siegelabschnitte des Siegelelements (100) hervorzurufen, wobei die inneren Siegelabschnitte die im Wesentlichen flüssigkeitsdichte Siegelbeziehung mit dem chirurgischen Gegenstand (I) beibehalten.

2. Chirurgisches Zugangsgerät nach Anspruch 1, wobei das erste und das zweite Kabelende (112, 114) mit dem entsprechenden ersten und zweiten radialen Abschnitt (116, 118) des Siegelelements (100) an Stellen (120, 122) verbunden sind, die den inneren Siegelabschnitten benachbart sind.

3. Chirurgisches Zugangsgerät nach Anspruch 2, umfassend ein erstes und ein zweites Kabelelement (110A, 110B), wobei jedes Kabelelement (110A, 110B) Kabelenden (112A, 112B, 114A, 114B) aufweist, die mit beabstandeten radialen Abschnitten (116A, 116B, 118A, 118B) des Siegelelements (100) verbunden sind.

4. Chirurgisches Zugangsgerät nach Anspruch 1, wobei das erste Kabelende (112) mit dem ersten radialen Abschnitt (116) an einer ersten Stelle (120) verbunden ist, und das zweite Kabelende (114) mit dem zweiten radialen Abschnitt (118) an einer zweiten Stelle (122) verbunden ist, wobei die erste und die zweie Stelle (120, 122) im Wesentlichen der Öffnung (108) benachbart sind.

5. Chirurgisches Zugangsgerät nach Anspruch 1, wobei das Siegelelement (100) mindestens einen Kanal (124) definiert, der konfiguriert ist, um mindestens teilweise das mindestens eine Kabelelement (110) aufzunehmen.

6. Chirurgisches Zugangsgerät nach Anspruch 5, wobei der mindestens eine Kanal (124) im Siegelelement (100) definiert ist.

7. Chirurgisches Zugangsgerät nach Anspruch 6, wobei der mindestens eine Kanal (124) in einer Außenfläche (106) des Siegelelements (100) definiert ist.

8. Chirurgisches Zugangsgerät nach Anspruch 1, wobei das mindestens eine Kabelelement (110) eine Länge definiert, die während der Verwendung des chirurgischen Siegelelements (110) im Wesentlichen gleich bleibt.

9. Chirurgisches Zugangsgerät nach Anspruch 1, wobei das mindestens eine Kabelelement (110) aus einem im Wesentlichen nicht starren Material gebildet ist.

10. Chirurgisches Zugangsgerät nach Anspruch 1, wobei das Siegelelement (100) aus einem elastomeren Material gebildet ist, so dass das Siegelelement nach dem Einführen bzw. der Entfernung des chirurgischen Gegenstands elastisch von einem ersten in einen zweiten Zustand übergeht.

11. Chirurgisches Zugangsgerät nach Anspruch 1, wobei die Öffnung (108) des Siegelelements (100) einen ersten Durchmesser (D₁) im ersten Zustand und einen zweiten Durchmesser (D₂) im zweiten Zustand definiert.

12. Chirurgisches Zugangsgerät nach Anspruch 11, wobei das mindestens eine Kabelelement (110) konfiguriert ist, um die Öffnung (108) des Siegelelements (100) bei einer seitlichen Betätigung des chirurgischen Gegenstands (I), der **dadurch** eingeführt ist, zu verschieben, wobei die Öffnung (108) in der Richtung der seitlichen Betätigung verschoben wird, so dass der zweite Durchmesser (D₂) der Öffnung (108) im Wesentlichen konstant bleibt.

13. Chirurgisches Zugangsgerät nach Anspruch 1, wobei das Zugangselement (10) eine Kanüle (16) umfasst, die eine Längsöffnung definiert, die für den Durchgang des chirurgischen Gegenstands dimensioniert ist.

14. Chirurgisches Zugangsgerät nach Anspruch 13, weiter umfassend ein Gehäuse (14), das an die Kanüle gekoppelt ist, wobei das Gehäuse konfiguriert ist, um das Siegelelement (100) aufzunehmen und mindestens einen Kabelkanal (20) definiert, wobei der mindestens eine Kabelkanal (20) konfiguriert ist, um die Verschiebung des mindestens eines Kabelelements (110) während der seitlichen Betätigung des chirurgischen Gegenstands im Siegelelement (110) zu ermöglichen.

## Revendications

1. Appareil d'accès chirurgical pour un objet chirurgical, qui comprend :
un élément d'accès (10) définissant un axe longitudinal et ayant un passage longitudinal pour la réception et le passage d'un objet chirurgical (I),
un élément de joint (100) monté sur l'élément d'accès (10), l'élément de joint (100) ayant des parties de joint internes définissant une ouverture (108) afin de recevoir de manière amovible l'objet chirurgical (I) dans une relation sensiblement étanche au fluide avec celui-ci ; et **caractérisé par**
au moins un élément de câble (110), ledit au moins un élément de câble (110) ayant une première extrémité de câble (112) raccordée à une première section radiale (116) de l'élément de joint (100) et une seconde extrémité de câble (114) raccordée à une seconde section radiale (118) de l'élément de joint (100), déplacé depuis la première section radiale (116), dans lequel la première et la seconde extrémités sont dans une relation sensiblement diamétralement opposée, la première et la seconde extrémités de câble (112, 114) étant adaptées pour être déplacées latéralement par rapport à l'axe longitudinal, pendant un mouvement latéral décalé de l'objet chirurgical, afin de provoquer ainsi un déplacement latéral correspondant des parties de joint internes de l'élément de joint (100), moyennant quoi les parties de joint interne maintiennent la relation de scellement sensiblement étanche au fluide avec l'objet chirurgical (I).

2. Appareil d'accès chirurgical selon la revendication 1, dans lequel la première et la seconde extrémités de câble (112, 114) sont raccordées aux première et seconde sections radiales respectives (116, 118) de l'élément de joint (100) aux emplacements (120, 122) adjacents aux parties de joint interne.

3. Appareil d'accès chirurgical selon la revendication 2, comprenant un premier et un second éléments de câble (110A, 110B), chaque élément de câble (110A, 110B) ayant des extrémités de câble (112A, 112B, 114A, 114B) raccordées à des sections radiales espacées (116A, 116B, 118A, 118B) de l'élément de joint (100).

4. Appareil d'accès chirurgical selon la revendication 1, dans lequel la première extrémité de câble (112) est raccordée à la première section radiale (116) à un premier emplacement (120) et la seconde extrémité de câble (114) est raccordée à la seconde section radiale (118) au second emplacement (122), le premier et le second emplacements (120, 122) étant sensiblement adjacents à l'ouverture (108).

5. Appareil d'accès chirurgical selon la revendication 1, dans lequel l'élément de joint (100) définit au moins un canal (124) configuré pour recevoir au moins partiellement ledit au moins un élément de câble (110).

6. Appareil d'accès chirurgical selon la revendication 5, dans lequel ledit au moins un canal (124) est défini dans l'élément de joint (100).

7. Appareil d'accès chirurgical selon la revendication 6, dans lequel ledit au moins un canal (124) est défini sur une surface extérieure (106) de l'élément de joint (100).

8. Appareil d'accès chirurgical selon la revendication 1, dans lequel ledit au moins un élément de câble (110) définit une longueur qui reste sensiblement constante pendant l'utilisation de l'élément de joint chirurgical (110).

9. Appareil d'accès chirurgical selon la revendication 1, dans lequel ledit au moins un élément de câble (110) est formé d'un matériau sensiblement non rigide.

10. Appareil d'accès chirurgical selon la revendication 1, dans lequel l'élément de joint (100) est formé d'un matériau élastomère, de sorte que l'élément de joint passe de manière souple entre une première et une seconde conditions, lors de l'insertion et de l'enlèvement respectifs de l'objet chirurgical.

11. Appareil d'accès chirurgical selon la revendication 1, dans lequel l'ouverture (108) de l'élément de joint (100) définit un premier diamètre (D1) dans la première condition et un second diamètre (D2) dans la seconde condition.

12. Appareil d'accès chirurgical selon la revendication 11, dans lequel ledit au moins un élément de câble (110) est configuré pour déplacer l'ouverture (108) de l'élément de joint (100), lors de la manipulation latérale de l'objet chirurgical (I) inséré à travers lui, l'ouverture (108) étant déplacée dans le sens de la manipulation latérale, de sorte que le second diamètre (D2) de l'ouverture (108) reste sensiblement constant.

13. Appareil d'accès chirurgical selon la revendication 1, dans lequel l'élément d'accès (10) comprend une canule (16) définissant une ouverture longitudinale dimensionnée pour le passage de l'objet chirurgical.

14. Appareil d'accès chirurgical selon la revendication 13, comprenant en outre un logement (14) raccordé à la canule, le logement étant configuré pour recevoir l'élément de joint (100) et définissant au moins un conduit de câble (20), ledit au moins un conduit de câble (20) étant configuré pour permettre le déplacement dudit au moins un élément de câble (110), pendant la manipulation latérale de l'objet chirurgical dans l'élément de joint (110).
